# EUROPEAN PATENT APPLICATION

(11) **EP 4 393 956 A1**
(43) Date of publication of application: **03.07.2024**
(21) Application number: 22861742.9
(22) Date of filing: 25.08.2022
(51) Int. Cl.: C07K 16/30, A61K 47/68, A61P 35/00, G01N 33/574, A61K 39/00

(54) **NOVEL ANTI-MUC1 ANTIBODY AND USE THEREOF**

(30) Priority: 27.08.2021 KR 20210113712
(71) Applicant: Peptron, Inc., Daejeon 34054 (KR)
(72) Inventor: CHOI, Hoil, Daejeon 34054 (KR)
(74) Representative: Cabinet Becker et Associés
(86) International application number: PCT/KR2022/012764
(87) International publication number: WO 2023/027534

(57) **Abstract**

The present invention relates to a novel antibody and a use thereof, and more specifically, to: an antibody-drug conjugate or a bispecific antibody comprising the antibody produced by substituting an existing antibody sequence or an antigen-binding fragment thereof, wherein the antibody does not have any glycation-related substances generated in a production process; a pharmaceutical composition comprising the antibody-drug conjugate or bispecific antibody for preventing or treating cancer; a nucleic acid encoding the antibody or an antigen-binding fragment thereof; a vector and a host cell comprising the nucleic acid; and a method for preparing an antibody or an antigen-binding fragment thereof.

## Description

### [Technical Field]

The present invention relates to an anti-MUC1 antibody that specifically binds to Mucin 1 (MUC1) and a use thereof, wherein the anti-MUC1 antibody or an antigen-binding fragment thereof has an amino acid sequence to increase purity during production. Further, the present invention relates to an antibody-drug conjugate or a bispecific antibody including the antibody or an antigen-binding fragment thereof, a pharmaceutical composition including the antibody-drug conjugate or bispecific antibody for preventing or treating cancer, a nucleic acid encoding the antibody or an antigen-binding fragment thereof, a vector and a host cell comprising the nucleic acid, and a method for producing an anti-MUC1 antibody or an antigen-binding fragment thereof using the vector and the host cell.

### [Background Art]

Mucin 1 (MUC1) is a transmembrane glycoprotein containing a plurality of glycated extracellular domains. The MUC1 has a length of 200 to 500 nm from the cell surface and is located in the apical membrane of normal epithelial cells. The MUC1 is expressed in glandular or luminal epithelial cells of many organs, such as breast, stomach, esophagus, pancreas, urethra, lung, kidney, and gallbladder. In normal tissues, the negatively charged carbohydrates of MUC1 form physical barriers that protect the basal epithelium from dehydration, pH changes, pollen, and microorganisms. A MUC1 gene encodes a single transcript. After translation, the MUC1 is auto cleaved at a GSVVV motif located within sea urchin sperm protein enterokinase and an agrin (SEA) domain. The MUC1 gene consists of two peptide fragments of an N-terminal subunit (MUC1-N) and a C-terminal subunit (MUC1-C).

After MUC1 is first expressed, in the MUC1, a region bound to a cell and a region that may be released outside the cell are bound with each other through noncovalent interaction. In this case, cleavage is caused by an enzyme called "Sheddase". A MUC1 complex is isolated by stimulation of cytokines such as IFNγ and TNF-α. MUC1-N is released by enzymes including a TNF-α converting enzyme (TACE) and a matrix metallo-protease (MMP). These enzymes cleave an extracellular domain of MUC1-C into two fragments. As cancer progresses, these extracellular fragments are isolated from the cancer cells and float in the body's blood, whereas the fragments bound to the cells exist in a continuously connected to the cancer cells.

The MUC1 is important for the growth of cancer cells, which is because the MUC1 plays a critical role in cancer cell proliferation by transmitting a continuous cell proliferation signal through binding to cell membrane proteins related to cancer cell proliferation present in other cancer cells. In addition, this region always shares the same fate as the cancer cell until the growth and death to be a good target for cancer detection and a critical biomarker capable of eliminating cancer. In addition, unlike other regions of MUC1, this region is known as only a region that does not undergo glycosylation and was considered as a region that shows a clear difference in distinguishing MUC1 in normal cells from MUC1 in cancer cells. Accordingly, the present inventors found that a cell-binding region of MUC1 shared the same fate as cancer, and that this fragment capable of distinguishing MUC1 in normal cells from MUC1 in cancer cells was an optimal antigen for an antibody and developed an antibody targeting a MUC1-C terminal.

The information presented in the background art is just for the purpose of improving the understanding of the background of the present invention. Accordingly, information forming the prior arts known to those skilled in the art to which the present invention pertains may not be included.

### [Disclosure]

### [Technical Problem]

An object of the present invention is to provide an anti-MUC1 antibody or an antigen-binding fragment thereof that specifically binds to a "cell-binding region" of MUC1 and has reduced glycation at a specific residue. An object of the present invention is to provide an anti-MUC1 antibody or an antigen-binding fragment thereof that specifically binds to a "cell-binding region" of MUC1 and has reduced glycation at a specific residue.

Another object of the present invention is to provide an antibody-drug conjugate in which a drug is conjugated with the antibody or an antigen-binding fragment thereof, a bispecific antibody including the antibody or an antigen-binding fragment thereof, or CAR-T, CAR-natural killer cell (NK) and/or CAR-Macrophage (MA) as an immune cell including a chimeric antigen receptor (CAR) including the antibody or an antigen-binding fragment thereof.

Yet another object of the present invention is to provide a composition for use in preventing or treating cancer and a method for treating cancer, the composition including the anti-MUC1 antibody or an antigen-binding fragment thereof, the antibody-drug conjugate, or the bispecific antibody.

Yet another object of the present invention is to provide a cancer diagnostic composition and a diagnostic method including the anti-MUC1 antibody or an antigen-binding fragment thereof.

Yet another object of the present invention is to provide a nucleic acid encoding the anti-MUC1 antibody or an antigen-binding fragment thereof, a vector and a host cell including the nucleic acid, and a method for preparing an anti-MUC1 antibody or an antigen-binding fragment thereof using the vector and the host cell.

### [Technical Solution]

In order to achieve the above object, an aspect of the present invention provides an antibody or an antigen-binding fragment thereof in which some amino acid residues are substituted, as an anti-MUC1 antibody or an antigen-binding fragment thereof that recognizes a polypeptide containing five or more contiguous amino acids within a C-terminal extracellular domain of MUC1.

Preferably, the anti-MUC1 antibody or an antigen-binding fragment thereof comprises six complementarity determining regions (CDRs). The anti-MUC1 antibody or an antigen-binding fragment thereof may include at least one sequence selected from the group consisting of a heavy chain CDR1 represented by SEQ ID NO: 1 (GYTFTSYWMH); a heavy chain CDR2 represented by SEQ ID NO: 2 (YINPGTGYIEYNQKFKD); a heavy chain CDR3 represented by SEQ ID NO: 3 (STAPFDY); a light chain CDR1 represented by SEQ ID NO: 4 (XASQDIXSYLS); a light chain CDR2 represented by SEQ ID NO: 5 (YATRLAD); and a light chain CDR3 represented by SEQ ID NO: 6 (LQYDESPYT), and a lysine (K) residue included in the light chain CDR sequence may be substituted with another amino acid.

Another aspect of the present invention provides a hybridoma for producing the anti-MUC1 antibody.

Yet another aspect of the present invention provides an antibody-drug conjugate or a bispecific antibody or CAR-T, CAR-NK and/or CAR-MA as an immune cell including a chimeric antigen receptor (CAR) comprising the anti-MUC1 antibody or an antigen-binding fragment thereof.

Yet another aspect of the present invention provides a pharmaceutical composition for use in preventing or treating cancer and a method for treating cancer comprising the anti-MUC1 antibody or an antigen-binding fragment thereof, the antibody-drug conjugate, or the bispecific antibody.

Yet another aspect of the present invention provides a cancer diagnostic composition and a diagnostic method including the anti-MUC 1 antibody or an antigen-binding fragment thereof.

Yet another aspect of the present invention provides a nucleic acid encoding the anti-MUC1 antibody or an antigen-binding fragment thereof, a vector and a host cell including the nucleic acid, and a method for preparing an anti-MUC1 antibody or an antigen-binding fragment thereof using the vector and the host cell.

Mucin 1 (MUC1) is generally expressed on one side (apical membrane) of a normal epithelial cell and is also abnormally expressed at high levels in various cancer cells. MUC1 expressed in cancer cells has a reduced degree of glycation and is evenly expressed on the entire surface of the cells and involved in promoting the proliferation, invasion, metastasis, and angiogenesis of cancer cells. The cancer-specific Mucin 1 (MUC1) expressed in cancer cells is generally expressed on one side (apical membrane) of normal epithelial cells, and also abnormally expressed at high levels in various cancer cells. The MUC1 expressed in cancer has a reduced degree of glycation and is evenly expressed on the entire surface of the cells and involved in promoting the proliferation, invasion, metastasis, and angiogenesis of cancer cells, so that the MUC1 expressed in cancer cells is a target for cancer-specific treatment.

In particular, a MUC1 C-terminal subunit (MUC1-C) domain is located in the transmembrane or cytoplasm, and after the extracellular domain of MUC1 is cleaved by an enzyme, a MUC1-C terminal region (extracellular domain) remains on the cell surface. The present invention provides a novel antibody that specifically binds to a MUC1 C-terminal targeting the same, and particularly, a MUC1-C antibody or an antigen-binding molecule thereof in which some lysine (K) residues included in the CDR of the antibody are substituted, thereby reducing glycation of the corresponding region.

That is, in one aspect, the present invention relates to an anti-MUC1 antibody or an antigen-binding fragment thereof that specifically binds to MUC1.

More specifically, the antibody or an antigen-binding fragment of the present invention is an anti-MUC1 antibody or an antigen-binding fragment thereof that recognizes a polypeptide containing five or more contiguous amino acids in the C-terminal extracellular domain of MUC1 and may include substitution of one or more amino acid residues in the CDR sequence of the antibody or an antigen-binding fragment thereof.

As used herein, "antibody" is a general term for substances produced by stimulation of antigens in the immune system, and types thereof are not particularly limited. The antibody is an immunoglobulin molecule that is immunologically reactive with a specific antigen and refers to a protein molecule that acts as a receptor that specifically recognizes the antigen and may include all of a polyclonal antibody and a monoclonal antibody, and a whole antibody and antibody fragments. The antibody may be non-naturally produced, for example, recombinantly or synthetically produced. The antibody may be an animal antibody (e.g., mouse antibody, etc.), a chimeric antibody, a humanized antibody, or a human antibody. The antibody may be a monoclonal antibody. In addition, unless otherwise specified, it may be understood that the antibody also includes an antigen-binding fragment of an antibody having antigen-binding ability.

As used herein, "complementarity-determining regions (CDR)" refer to regions that impart binding specificity to the antigen in variable regions of the antibody. The antigen-binding fragment of the antibody described above may be an antibody fragment comprising one or more of complementarity-determining regions.

In the present invention, the anti-MUC1 antibody may be produced from a hybridoma.

In addition, the anti-MUC1 antibody or an antigen-binding fragment thereof has complementarity determining regions CDR-H1, CDR-H2, CDR-H3, CDR-L1, CDR-L2 and CDR-L3 or heavy chain variable regions and light chain variable regions of the antibody produced from the hybridoma.

In an aspect, the anti-MUC1 antibody or an antigen-binding fragment thereof according to the present invention includes six complementarity-determining regions (CDRs). The antibody or an antigen-binding fragment thereof includes at least one sequence selected from the group consisting of a heavy chain CDR1 represented by SEQ ID NO: 1 (GYTFTSYWMH); a heavy chain CDR2 represented by SEQ ID NO: 2 (YINPGTGYIEYNQKFKD); a heavy chain CDR3 represented by SEQ ID NO: 3 (STAPFDY); a light chain CDR1 represented by SEQ ID NO: 4 (XASQDIXSYLS); a light chain CDR2 represented by SEQ ID NO: 5 (YATRLAD); and light chain CDR3 represented by SEQ ID NO: 6 (LQYDESPYT), and one or more amino acid residues in the antibody CDR may be substituted with other amino acids.

More specifically, the positions of the substituted amino acids in the antibody or an antigen-binding molecule of the present invention are amino acid residues at positions 1 and 7 in the light chain CDR sequence represented by SEQ ID NO: 4. That is, the amino acid residue to be substituted is substituted from lysine K24 or K30 of the light chain CDR of the anti-MUC1 antibody of the present invention. The amino acid to be substituted is not limited to the type thereof, but may be preferably one selected from the group consisting of arginine ^{®}, histidine (H), aspartic acid (D), or glutamic acid (G), which is an amino acid containing electrically polar side chains, or glycine (G), alanine (A), valine (V), methionine (M), phenylalanine (F), tyrosine (Y), tryptophan (W), leucine (L), or isoleucine (I), which is an amino acid containing hydrophobic side chains, and more preferably arginine ^{®} or glycine (G). As such, since post-translational modification by glycation of the residues does not occur due to the substitution of lysine residues in the CDR, proteome complexity is reduced to have an effect of preparing a high-purity antibody or antigen-binding fragment thereof.

In the present invention, the anti-MUC1 antibody or an antigen-binding fragment thereof may be characterized by recognizing or specifically binding to a polypeptide (epitope) containing 5, 7, 10, 12 or more, or preferably 15 or more amino acids in the MUC1 protein, specifically the C-terminal extracellular domain of the MUC1 protein. The antibody or an antigen-binding fragment thereof may be characterized by recognizing and/or specifically binding to an extracellular domain of the MUC1 protein, an SEA domain of the MUC1 protein, or a C-terminal extracellular domain of the MUC1 protein. As used herein, the term "MUC1-specific antibody" or "antibody specifically binding to MUC1" refers to an antibody that binds to MUC1 to cause inhibition of the biological activity of MUC1 and is used interchangeably with the "anti-MUC1 antibody." As used herein, the "anti-MUC1 antibody" may be an animal antibody (e.g., mouse antibody), a chimeric antibody (e.g., mouse-human chimeric antibody), or a humanized antibody, and may be a monoclonal antibody or a polyclonal antibody, for example, a monoclonal antibody. The anti-MUC1 antibody is a concept that includes both a polyclonal antibody and a monoclonal antibody, and preferably a monoclonal antibody, and may be in the form of an intact whole antibody. The whole antibody has a structure having two full-length light chains and two full-length heavy chains and a structure including constant regions, and each light chain is linked to the heavy chain by disulfide bonds.

The whole antibody of the anti-MUC1 antibody according to the present invention is a concept including IgA, IgD, IgE, IgM, and IgG forms, and IgG as a subtype includes IgG1, IgG2, IgG3, and IgG4.

The intact IgG-type antibody has a structure having two full-length light chains and two full-length heavy chains, and each light chain is linked to the heavy chain by disulfide bonds. The constant region of the antibody is divided into a heavy chain constant region and a light chain constant region, and the heavy chain constant region has gamma (γ), mu (µ), alpha (α), delta (δ), and epsilon (ε) types, and subclasses include gamma 1 (γ1), gamma 2 (y2), gamma 3 (y3), gamma 4 (y4), alpha 1 (α1) and alpha 2 (α2). The light chain constant region has kappa (κ) and lambda (λ) types.

As used herein, the term "heavy chain" is interpreted as meaning including all of a full-length heavy chain comprising a variable region domain VH including an amino acid sequence having a sufficient variable region sequence to impart specificity to an antigen, three constant region domains CH1, CH2 and CH3 and hinges, and fragments thereof. In addition, the term "light chain" is interpreted as meaning including all of a full-length light chain comprising a variable region domain VL including an amino acid sequence having a sufficient variable region sequence to impart specificity to an antigen and a constant region domain CL, and fragments thereof.

As used herein, the "complementarity determining region (CDR)" refers to an amino acid sequence of a hypervariable region of the heavy and light chains of immunoglobulin. The heavy and light chains may include three CDRs CDRH1, CDRH2, CDRH3 and CDRL1, CDRL2, CDRL3, respectively. The CDRs may provide key contact residues for the antibody binding to an antigens or epitope.

Meanwhile, the term "specifically binding" or "specifically recognizing" has the same meaning as commonly known to those skilled in the art and means that an antigen and an antibody specifically interact with each other to cause an immunological response.

The "antigen-binding fragment" of the anti-MUC 1 antibody according to the present invention refers to a fragment that has a function capable of binding to the antigen of the anti-MUC1 antibody, that is, MUC1, and corresponds to a concept including Fab, Fab', F(ab')2, scFv, (scFv)2, scFv-Fc, and Fv, and is used interchangeably with the same meaning as the "antibody fragment". For example, the antigen-binding fragment may be scFv, (scFv)2, Fab, Fab' or F(ab')2, but is not limited thereto. The Fab of the antigen binding fragments has a structure having variable regions of the light and heavy chains, a constant region of the light chain, and a first constant region (CH1) of the heavy chain and has one antigen binding site. The Fab' is different from Fab by having a hinge region containing one or more cysteine residues in a C-terminal of the heavy chain CH1 domain. The F(ab')2 antibody is produced when the cysteine residues in the hinge region of Fab' form disulfide bonds. The Fv is a minimal antibody fragment having only a heavy chain variable region and a light chain variable region, and a recombination technique for generating the Fv fragment is widely known in the art. The two-chain Fv has the heavy chain variable region and the light chain variable region linked by non-covalent bonds, and the single-chain Fv may generally form a dimer-like structure, such as the two-chain Fv because the variable region of the heavy chain and the variable region of the light chain are covalently linked to each other via a peptide linker or directly linked at a C-terminal. The antigen-binding fragments may be obtained using protease (for example, the whole antibody is restriction-cleaved with papain to obtain Fab and cleaved with pepsin to obtain an F(ab')2 fragment) or may be produced through genetic recombination technology.

As used herein, the term "hinge region" refers to a region included in the heavy chain of the antibody and means a region which is present between the CH1 and CH2 regions and functions to provide flexibility of the antigen binding site in the antibody.

The anti-MUC1 antibody may be a monoclonal antibody. The monoclonal antibody may be prepared according to methods well known in the art. For example, the monoclonal antibody may be prepared using a phage display method. Alternatively, the monoclonal antibody may be prepared as a mouse-derived monoclonal antibody by a conventional method using the anti-MUC1 antibody.

Meanwhile, individual monoclonal antibodies may be screened based on the binding ability to MUC1 using a typical Enzyme-Linked ImmunoSorbent Assay (ELISA) format. Conjugates may be tested for inhibitory activity through functional assays such as competitive ELISA or cell-based assays to test molecular interactions. Then, the respective affinities (Kd values) with MUC1 are tested for monoclonal antibody members selected based on strong inhibitory activity.

The scope of the anti-MUC1 antibody or an antigen-binding fragment thereof according to the present invention includes peptides and aptamers that have substantially the same binding ability and specificity to the MUC1 antigen while having at least one of CDR1 to CDR3 of the light and heavy chains included in the anti-MUC1 antibody or an antigen-binding fragment thereof according to the present invention.

In another aspect, the present invention relates to a hybridoma for producing the anti-MUC1 antibody. The present invention provides an anti-MUC1 antibody, or an antigen-binding fragment thereof produced by the hybridoma. As another example, the present invention provides an anti-MUC1 antibody or an antigen-binding fragment thereof including a heavy chain complementarity determining region (CDR-H1, CDR-H2, CDR-H3, or a combination thereof), a light chain complementarity determining region (CDR-L1, CDR-L2, CDR-L3, or a combination thereof), or a combination thereof of the anti-MUC1 antibody produced by the hybridoma; or a heavy chain variable region, a light chain variable region, or a combination thereof of the anti-MUC1 antibody produced by the hybridoma.

In this case, the complementarity determining region may be determined by all conventional methods, for example, by IMGT definition or Kabat definition, but is not limited thereto.

In the present invention, the anti-MUC1 antibody or an antigen-binding fragment thereof specifically recognizes the MUC1-C terminal extracellular domain, so that the anti-MUC1 antibody or an antigen-binding fragment thereof may act specifically on cancer or tumor cells which express more MUC1-C terminal extracellular domain and less glycated than normal cell, and may recognize/bind to a MUC1 protein expressed not only on one surface but also on the entire surface of the cell. In addition, the anti-MUC1 antibody or an antigen-binding fragment thereof not only binds to the MUC1 protein, especially the MUC1-C terminal extracellular domain, but also is internalized into the cell to effectively inhibit a MUC1-mediated pathway, thereby maximizing a pharmacological effect. In addition, the internalization characteristic of the anti-MUC1 antibody or an antigen-binding fragment thereof has an advantage of effectively delivering the conjugated drug into cells when applied as an antibody-drug conjugate (ADC).

In yet another aspect, the present invention relates to an immune cell including a chimeric antigen receptor (CAR) containing an anti-MUC1 antibody or an antigen-binding fragment thereof according to the present invention, more specifically a CAR-T, CAR-natural killer cell (NK) and/or CAR-macrophage (MA) cell, and a therapeutic agent including the immune cell. The form of the CAR-T, CAR-NK, CAR-MA or the anti-MUC1 antibody or an antigen-binding fragment thereof included therein is preferably scFv but is not limited thereto.

In yet another aspect, the present invention relates to an antibody-drug conjugate (ADC) in which the drug is conjugated with the anti-MUC1 antibody or an antigen-binding fragment thereof.

In the antibody-drug conjugate (ADC), an anti-cancer drug needs to stably bind to an antibody until delivering the anti-cancer drug to a target cancer cell. The drug delivered to the target needs to be released from the antibody to induce the death of the target cell. To this end, the drug needs to have sufficient cytotoxicity to induce the death of the target cell when released from the target cell while stably binding to the antibody.

In the present invention, the antibody-drug conjugate may comply with a technique well known in the art to which the present invention pertains.

In the present invention, the antibody-drug conjugate may be characterized in that the antibody or an antigen-binding fragment thereof binds to a drug through a linker.

In yet another aspect, the present invention relates to a bispecific antibody including the anti-MUC1 antibody or an antigen-binding fragment thereof. The bispecific antibody means a form in which in two arms of the antibody, one arm includes the anti-MUC1 antibody or an antigen-binding fragment thereof according to the present invention, and the other arm includes an antibody specific to an antigen other than MUC1, preferably a cancer-related antigen or immune checkpoint protein antigen, or an antibody or antigen-binding fragment thereof specifically binding to an immune effector cell-related antigen.

The antigen binding to the antibody other than the anti-MUC1 antibody included in the bispecific antibody according to the present invention may be preferably selected from Her2, EGFR, VEGF, VEGF-R, CD-20, MUC16, CD30, CD33, CD52, PD-1, PD-L1, CTLA4, BTLA4, EphB2, E-selectin, EpCam, CEA, PSMA, PSA, ERB3, c-MET, etc. as the cancer-related antigen or immune checkpoint protein antigen, and selected from TCR/CD3, CD16(FcyRIIIa) CD44, CD56, CD69, CD64(FcγRI), CD89, CD11b/CD18(CR3), etc. as the immune effector cell-related antigen, but is not limited thereto.

In yet another aspect, the present invention relates to a pharmaceutical composition for use in preventing and/or treating MUC1-related disease, including the anti-MUC1 antibody or an antigen-binding fragment thereof, and an antibody-drug conjugate, a bispecific antibody, or an immune cell including a chimeric antigen receptor (CAR) including the anti-MUC1 antibody or an antigen-binding fragment thereof.

The MUC1-related diseases may be diseases associated with expression or overexpression of MUC1, expression of MUC1 on all surfaces of cells, and/or decreased glycation of the MUC1 protein compared to normal cells, for example, cancer. The normal cells may be non-tumor cells. Accordingly, the MUC1-related disease is preferably cancer or tumor but is not limited thereto.

The term "cancer" or "tumor" refers to or means a physiological condition in mammals typically characterized by uncontrolled cell growth/proliferation.

Cancers or tumors that may be treated with the composition of the present invention are not particularly limited and include both solid cancer and hematological cancer. Examples of these cancers may be selected from the group consisting of skin cancer such as melanoma, liver cancer, hepatocellular carcinoma, hepatocellular carcinoma, stomach cancer, breast cancer, lung cancer, ovarian cancer, bronchial cancer, nasopharyngeal cancer, laryngeal cancer, pancreas cancer, bladder cancer, colorectal cancer, colon cancer, pancreatic cancer, cervical cancer, brain cancer, prostate cancer, bone cancer, skin cancer, thyroid cancer, parathyroid cancer, kidney cancer, esophageal cancer, biliary tract cancer, testicular cancer, rectal cancer, head and neck cancer, cervical spine cancer, ureteral cancer, osteosarcoma, neuroblastoma, fibro sarcoma, rhabdomyosarcoma, astrocytoma, nerve blastoma, glioma, acute myeloid leukemia (AML), acute lymphoblastic leukemia (ALL), adult T-cell leukemia, chronic lymphocytic leukemia (CLL), hairy cell leukemia, myelodysplasia, myeloproliferative disorder, chronic myeloid leukemia (CML), myelodysplastic syndrome (MDS), human T cell leukemia virus type 1 (HTLV-1) leukemia, mastocytosis, acute lymphoblastic leukemia, lymphoma, non-Hodgkin's lymphoma, Hodgkin's lymphoma, multiple myeloma, or solitary myeloma, but are not limited thereto.

More specifically, the cancer is characterized by expression of the MUC1 protein, and may include breast cancer, pancreatic cancer, prostate cancer, lung cancer, thyroid cancer, stomach cancer, ovarian cancer, colon cancer, liver cancer, gallbladder cancer, kidney cancer, cervical cancer, or bladder cancer, but is not limited thereto. The cancer may be a primary cancer or a metastatic cancer.

The MUC1-related diseases may be Non-Alcoholic SteatoHepatitis (NASH) or TGF-β-mediated immune diseases but is not limited thereto. In an embodiment of the present invention, in the pharmaceutical composition, method and use for preventing and/or treating cancer, the anti-MUC1 antibody or an antigen-binding fragment thereof may be provided as a single active ingredient, administered in combination with a cytotoxic substance such as an anticancer agent, or provided in the form of an antibody-drug conjugate (ADC) conjugated with a cytotoxic substance such as an anticancer agent.

In addition, the anti-MUC1 antibody or an antigen-binding fragment thereof according to the present invention, and the pharmaceutical composition containing the anti-MUC1 antibody or an antigen-binding fragment thereof may be used in combination with conventional therapeutic agents. That is, the anti-MUC1 antibody or an antigen-binding fragment thereof according to the present invention, and the pharmaceutical composition containing the anti-MUC1 antibody or an antigen-binding fragment thereof may be administered simultaneously or sequentially with conventional therapeutic agents such as anticancer agents.

In yet another aspect, the present invention relates to a method for preventing and/or treating MUC1-related disease, including administering a therapeutically effective amount of the anti-MUC1 antibody or an antigen-binding fragment thereof or the antibody-drug conjugate to a patient in need of prevention and/or treatment of the MUC1-related disease. The prevention and/or treatment method may further include identifying the patient in need of prevention and/or treatment of the disease before the administration step.

The use of the antibody conjugate for local delivery of the drug in the composition allows the drug to be delivered to cells expressing the antigen targeted with the anti-MUC1 antibody.

The pharmaceutical composition may further comprise a pharmaceutically acceptable carrier. The pharmaceutically acceptable carrier is generally used in preparation of the drug and may be at least one selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like, but is not limited thereto. The pharmaceutical composition may further comprise at least one selected from the group consisting of a diluent, an excipient, a lubricant, a wetting agent, a sweetener, a flavoring agent, an emulsifier, a suspending agent, a preservative, etc., which are commonly used in the preparation of the pharmaceutical composition.

The pharmaceutical composition may be administered orally or parenterally. In the case of the parenteral administration, the pharmaceutical composition may be administered by intravenous injection, subcutaneous injection, intramuscular injection, intraperitoneal injection, endothelial administration, intranasal administration, intrapulmonary administration, intrarectal administration, lesion site local administration, or the like.

In the oral administration, since a protein or peptide is digested, the oral composition may be formulated so as to coat an active agent or to be protected from decomposition at the stomach. In addition, the composition may be administered by any device capable of moving the active substance to a target cell.

The content or dose of the anti-MUC1 antibody or an antigen-binding fragment thereof in the pharmaceutical composition may be variously prescribed by factors, such as a formulation method, an administration type, the age, weight, and sex of a patient, a pathological condition, food, an administration time, an administration interval, an administration route, an excretion rate, and response susceptibility. For example, a daily dose of the anti-MUC1 antibody or an antigen-binding fragment thereof may be in the range of 0.001 to 1000 mg/kg, specifically 0.01 to 100 mg/kg, more specifically 0.1 to 50 mg/kg, and much more specifically 0.1 to 20 mg/kg but is not limited thereto. The daily dose may be formulated as a single preparation in a unit dose form, formulated in appropriate portions, or prepared to be introduced in a multi-dose container.

The pharmaceutical composition may be formulated in the form of solutions, suspensions, syrups or emulsions in oils or aqueous media or in the form of extracts, powders, powders, granules, tablets, or capsules, and may further include a dispersant or a stabilizer for formulation.

The patient to be administered with the pharmaceutical composition may be mammals, including primates including humans and monkeys, and rodents including mice and rats.

As used herein, the treatment of cancer may refer to any anticancer action that prevents, alleviates, or improves the worsening of cancer symptoms, such as inhibiting the proliferation of cancer cells, killing cancer cells, or inhibiting metastasis, or partially or completely extinguishes cancer.

The anti-MUC1 antibody or an antigen-binding fragment thereof specifically binds to the MUC1 protein, especially the MUC1-C-terminal extracellular domain to detect or identify the MUC1 protein or the MUC1-C-terminal extracellular domain. Accordingly, in yet another aspect, the present invention relates to a method for detecting MUC1 including treating the anti-MUC1 antibody or an antigen-binding fragment thereof to a composition and a biological sample for detecting a MUC1 protein, for example, a MUC1-C-terminal extracellular domain including the anti-MUC1 antibody or an antigen-binding fragment thereof.

The detection method may further comprise identifying an antigen-antibody reaction, after the treating step. In the detection method, when the antigen-antibody reaction is detected, it may be determined (judged) that MUC1, for example, a MUC1-C-terminal extracellular domain is present in the biological sample. Accordingly, the detection method may further include determining that MUC1 is present in the biological sample when the antigen-antibody reaction is detected, after the identifying step. The biological sample may be selected from the group consisting of (isolated) cells, tissues, body fluids, cultures thereof, etc. obtained from mammals, such as humans (e.g., cancer patients).

The expression of the MUC1 protein, such as the C-terminal region of the MUC1 protein (or SEA domain or MUC1-C-terminal extracellular domain of the MUC1 protein), is associated with several diseases, such as cancer. Thus, in yet another aspect, the present invention relates to a composition for use in detecting or diagnosing MUC1 protein-related disease such as cancer, or a method for detecting or diagnosing cancer or a method for providing information for detection or diagnosis, including treating (administering) the anti-MUC1 antibody or an antigen-binding fragment thereof to a biological sample isolated from a subject.

The detection or diagnosis method may further comprise identifying an antigen-antibody reaction, after the treating step. In the method, when the antigen-antibody reaction is detected, it may be determined that the biological sample or the patient from which the biological sample is derived has MUC1-related disease, such as cancer. Accordingly, the method may further include determining the biological sample or the patient as the MUC1-related disease patient, such as a cancer patient, when the antigen-antibody reaction is detected, after the identifying step. The biological sample may be selected from the group consisting of (isolated) cells, tissues, body fluids, cultures thereof, etc. obtained from mammals, such as humans (e.g., cancer patients).

The identifying of the antigen-antibody reaction may be performed through various methods known in the art. For example, the antigen-antibody reaction may be measured by conventional enzymatic reactions, fluorescence, luminescence and/or radiation detection, and specifically, may be measured by a method selected from the group consisting of immunochromatography, immunohistochemistry, enzyme linked immunosorbent assay (ELISA), radioimmunoassay (RIA), enzyme immunoassay (EIA), fluorescence immunoassay (FIA), luminescence immunoassay (LIA), Western blotting, microarray, immunoprecipitation analysis, etc., but is not limited thereto.

In this case, the anti-MUC1 antibody or an antigen-binding fragment thereof may further include a labeling substance. The labeling substance may be one or more selected from the group consisting of radioisotopes, fluorescent substances, chromogens, dyeing substances, etc.

The labeling substance may be bound (linked) to the antibody or an antigen-binding fragment thereof by a conventional method (e.g., chemical bonds such as covalent bonds, coordination bonds, or ionic bonds). The binding of the antibody (or antigen-binding fragment) and the labeling substance may be performed according to techniques well known in the art to which the present invention pertains.

In yet another aspect, the present invention relates to a nucleic acid encoding an anti-MUC1 antibody according to the present invention. The nucleic acid used in the present invention may exist in a cell and a cell lysate, or also exist in a partially purified or substantially pure form. The nucleic acid is "isolated" or "substantially pured" when purified or released from other cellular components or other contaminants, for example, nucleic acids or proteins of other cells by standard techniques including alkali/SDS treatment, CsCl banding, column chromatography, agarose gel electrophoresis and others well known in the art. The nucleic acid of the present invention may be, for example, DNA or RNA, and may or not include an intron sequence.

In yet another aspect, the present invention relates to a recombinant expression vector comprising the nucleic acid. For expression of the anti-MUC1 antibody or an antigen-binding fragment thereof according to the present invention, DNA encoding partial or full-length light and heavy chains may be obtained by a standard molecular biology technique (e.g., PCR amplification or cDNA cloning using hybridomas expressing a target antibody), and the DNA may be "operably linked" to transcriptional and translational control sequences to be inserted into an expression vector.

As used herein, the term "operably linked" may mean that a gene encoding the antibody is ligated into a vector so that the transcriptional and translational control sequences in the vector perform an intended function of regulating transcription and translation of the antibody gene.

The expression vector and the expression control sequences are selected to be compatible with the host cell for expression to be used. The light chain gene of the antibody and the heavy chain gene of the antibody are inserted into separate vectors, or both the genes are inserted into the same expression vector.

The antibody is inserted into the expression vector by a standard method (e.g., ligation of an antibody gene fragment and a complementary restriction enzyme site on the vector, or blunt end ligation if there is no restriction enzyme site). In some cases, the recombinant expression vector may encode a signal peptide that facilitates the release of antibody chains from the host cell. The antibody chain gene may be cloned into a vector so that the signal peptide is linked to an amino terminal of the antibody chain gene according to a frame. The signal peptide may be an immunoglobulin signal peptide or a heterologous signal peptide (i.e., a signal peptide derived from a protein other than immunoglobulin). In addition, the recombinant expression vector has a regulatory sequence that controls the expression of the antibody chain gene in the host cell. The "regulatory sequence" may include a promoter, an enhancer, and other expression control elements (e.g., polyadenylation signal) that control the transcription or translation of the antibody chain gene. Those skilled in the art may recognize that a design of the expression vector may vary by selecting different regulatory sequences depending on factors such as the selection of a host cell to be transformed, the expression level of a protein, etc.

In yet another aspect, the present invention relates to a host cell comprising the nucleic acid or the vector. The host cell according to the present invention is preferably selected from the group consisting of animal cells, plant cells, yeast, prokaryotic cells, and insect cells, but is not limited thereto.

Specifically, the host cell according to the present invention may be a prokaryotic cell such as *Escherichia coli, Bacillus subtilis, Streptomyces* sp., *Pseudomonas* sp., *Proteus mirabilis,* or *Staphylococcus* sp. In addition, the host cell may be a eukaryotic cell, such as fungi such as *Aspergillus* sp., yeast such as *Pichia pastoris, Saccharomyces cerevisiae, Schizosaccharomyces* sp., and *Neurospora crassa,* other lower eukaryotic cells, and cells of higher eukaryotes such as cells from insects.

In addition, the host cell may be derived from plants or mammals. Preferably, the host cell is usable with monkey kidney cells (COS7), NSO cells, SP2/0, Chinese hamster ovary (CHO) cells, W138, baby hamster kidney (BHK) cells, MDCK, a myeloma cell line, HuT 78 cells, HEK293 cells, etc., but is not limited thereto. Particularly preferably, CHO cells may be used.

The nucleic acid or the vector is transfected into a host cell. The "transfection" may be used with a variety of different techniques commonly used to introduce an exogenous nucleic acid (DNA or RNA) into a prokaryotic or eukaryotic host cell, for example, electrophoresis, calcium phosphate precipitation, DEAE-dextran transfection, or lipofection. Various expression host/vector combinations may be used to express an anti-glypican 3 antibody according to the present invention. Expression vectors suitable for eukaryotic hosts are not limited to these, but include an expression regulatory sequence derived from SV40, bovine papillomavirus, adenovirus, adeno-associated virus, cytomegalovirus, and retrovirus. Expression vectors that may be used in bacterial hosts include bacterial plasmids obtained from *Escherichia coli,* such as pET, pRSET, pBluescript, pGEX2T, pUC vector, col E1, pCR1, pBR322, pMB9 and derivatives thereof; plasmids with a wider host range, such as RP4; phage DNA, which may be exemplified by a very variety of phage lambda derivatives such as λgt10, λgt11, and NM989, and other DNA phages such as M13 and filamentous single-stranded DNA phage. Expression vectors useful for yeast cells are 2°C plasmids and derivatives thereof. A vector useful for insect cells is pVL 941.

In yet another aspect, the present invention relates to a method for preparing an anti-MUC1 antibody or an antigen-binding fragment thereof according to the present invention, including expressing the anti-MUC1 antibody or an antigen-binding fragment thereof according to the present invention by culturing a host cell.

When the recombinant expression vector capable of expressing the anti-MUC1 antibody or an antigen-binding fragment thereof is introduced into a mammalian host cell, the antibody may be prepared by culturing the host cell for a period sufficient to express the antibody in the host cell, or more preferably for a period sufficient to release the antibody into a culture medium in which the host cell is cultured.

In some cases, the expressed antibody may be isolated from the host cell and purified to be uniform. The isolation or purification of the antibody may be performed by a conventional isolation and purification method used for the protein, for example, chromatography. The chromatography may include, for example, affinity chromatography including a protein A column and a protein G column, ion exchange chromatography, or hydrophobic chromatography. In addition to the chromatography, the antibody may be isolated and purified by additionally combining filtration, ultrafiltration, salting out, dialysis, etc.

### [Advantageous Effects]

According to the present invention, since an antibody or an antigen-binding fragment thereof specifically binding to MUC1 exhibits excellent affinity and binding force to MUC1, an antibody-drug conjugate, a bispecific antibody, or a chimeric antigen receptor (CAR) containing the antibody or an antigen-binding fragment thereof specifically binds to a MUC1 expressing cell, thereby effectively and specifically or selectively delivering a drug. Further, since post-translational modification in which the residues are glycated does not occur due to the substitution of some amino acid residues included in an antibody CDR sequence, proteome complexity is reduced to have an effect of preparing a high-purity antibody or antigen-binding fragment thereof. Therefore, an anti-MUC1 antibody and an antibody-drug conjugate according to the present invention can be usefully applied in the treatment of MUC1-related diseases, such as cancer.

### [Description of Drawings]

FIG. 1 illustrates results of confirming glycation peaks of an original form of PAb001 in which no point mutation occurred.
FIG. 2 illustrates results of confirming glycation peaks upon point mutagenesis in which lysine is substituted with arginine in a light chain K30 of PAb001.
FIG. 3 illustrates results of confirming glycation peaks upon point mutagenesis in which lysine are substituted with arginine in light chains K24 and K30 of PAb001.
FIG. 4 illustrates results of confirming glycation peaks upon point mutagenesis in which lysine is substituted with arginine in a light chain K30 of PAb001 and lysine is substituted with glycine in a light chain K24 of PAb001.
FIG. 5 illustrates results of confirming antigen-binding ability of PAb001 with point mutations.

### [Best Mode of the Invention]

Hereinafter, the present specification will be described in detail with reference to Examples for a specific description. However, the Examples according to the present specification may be modified in various different forms, and it is not interpreted that the scope of the present specification is limited to the Examples described in detail below. The Examples of the present specification will be provided for more completely explaining the present specification to those skilled in the art.

### Example 1. BAC analysis of anti-MUC1 antibody variants

In order to identify residues that caused glycation affecting purity using an anti-MUC1 antibody (PAb001) prepared in Korean Patent Registration No. 10-2127421, BAC analysis was performed on an original form and variants of PAb001. Residues of K24 and K30 in a light chain CDR of PAb001 were substituted with arginine (R) or glycine (G) and confirmed by performing experiments three times for each sample.

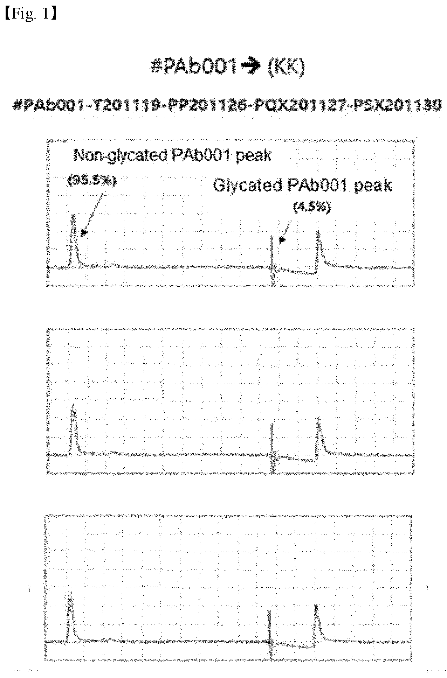

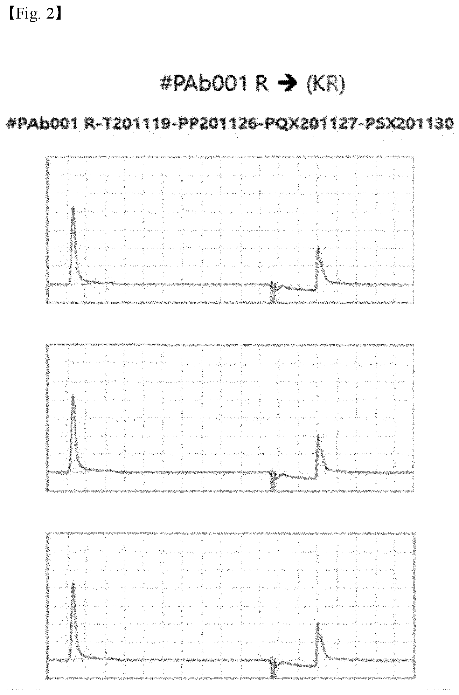

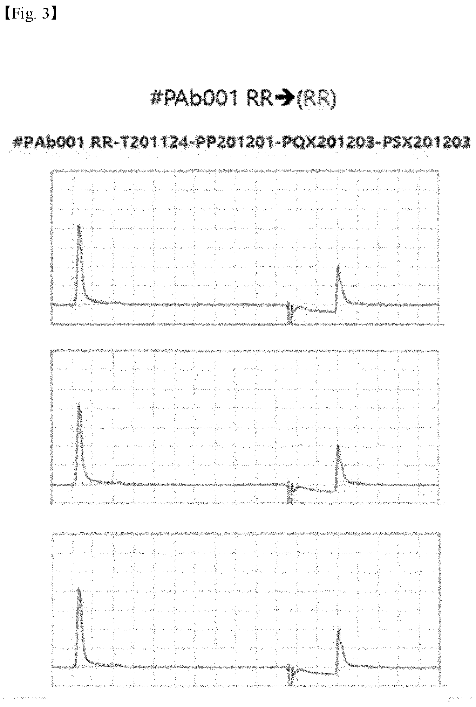

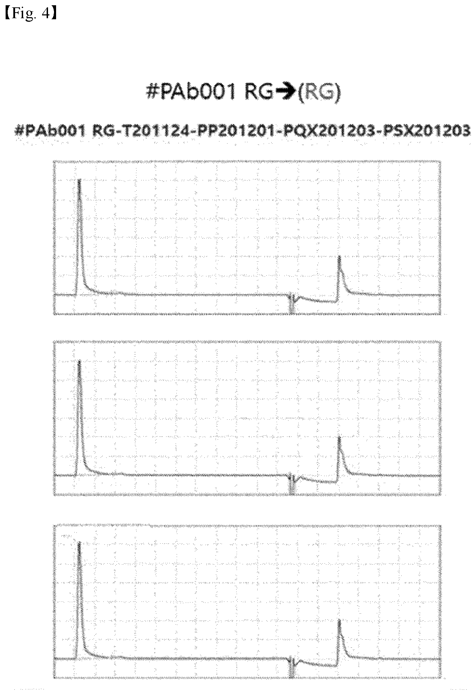

As a result, as shown in FIG. 1, peaks of both a non-glycation form and a glycation form were observed in an original form (KK) of PAb001. However, as shown in FIGS. 2 to 4, when K30 was substituted with arginine (R) or glycine (G), no glycation peak was observed. This shows that the glycation of PAb001 is changed by amino acid substitution of K30.

### Example 2. Antigen binding ability analysis of anti-MUC1 antibody variants

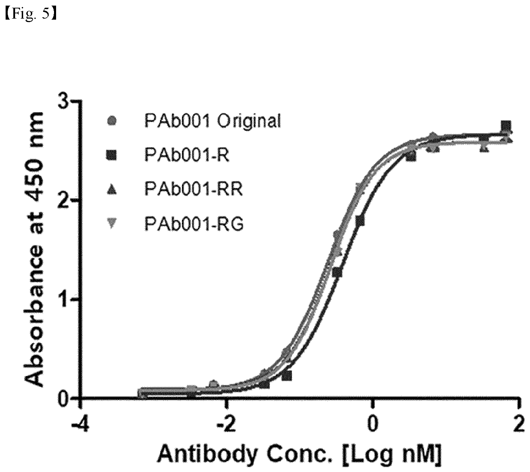

As a result of confirming the antigen binding ability of the original form and the variants of PAb001, as shown in FIG. 5, the tendencies were similar to have no significant difference. This shows that point mutation of K30 did not affect the antigen binding ability of the antibody.

As a result, it may be seen that when the point mutation is caused by substituting the light chain K30 of PAb001 with R or G, the glycation of the antibody is reduced without a functional difference in PAb001, making it possible to produce a high-purity antibody.

Hereinabove, the present invention has been described with reference to preferred examples thereof. It will be understood to those skilled in the art that the present disclosure may be implemented as modified forms without departing from an essential characteristic of the present disclosure. Therefore, the disclosed examples should be considered in an illustrative viewpoint rather than a restrictive viewpoint. The scope of the present disclosure is defined by the appended claims rather than by the foregoing description, and all differences within the scope of equivalents thereof should be construed as being included in the present disclosure.

### [Mode of the Invention]

As an aspect of the present invention, the present invention relates to an anti-MUC1 antibody or an antigen-binding fragment thereof that specifically binds to a polypeptide containing a contiguous amino acid sequence within a C-terminal extracellular domain of MUC1, and includes one or more of a heavy chain variable region including a heavy chain CDR1, a heavy chain CDR2, or a heavy chain CDR3; and a light chain variable region including a light chain CDR1, a light chain CDR2, or a light chain CDR3, wherein a lysine (K) residue included in the light chain CDR sequence is substituted with another amino acid.

In an aspect, the lysine (K) residue included in the light chain CDR sequence of the present invention is K24 or K30 of the light chain CDR.

In another aspect, the lysine (K) residue included in the light chain CDR sequence of the present invention is substituted with an amino acid selected from the group consisting of arginine (R), histidine (H), aspartic acid (D) or glutamic acid (G), glycine (G), alanine (A), valine (V), methionine (M), phenylalanine (F), tyrosine (Y), tryptophan (W), leucine (L), and isoleucine (I).

In yet another aspect, the heavy chain variable region of the present invention includes a heavy chain CDR1 represented by SEQ ID NO: 1, a heavy chain CDR2 represented by SEQ ID NO: 2, or a heavy chain CDR3 represented by SEQ ID NO: 3, and the light chain variable region includes a light chain CDR1 represented by SEQ ID NO: 4, a light chain CDR2 represented by SEQ ID NO: 5, or a light chain CDR3 represented by SEQ ID NO: 6.

As yet another aspect of the present invention, the present invention relates to an antibody-drug conjugate including the anti-MUC1 antibody or an antigen-binding fragment thereof.

As yet another aspect of the present invention, the present invention relates to a bispecific antibody including the anti-MUC 1 antibody or an antigen-binding fragment thereof.

As yet another aspect of the present invention, the present invention relates to a chimeric antigen receptor (CAR) including the anti-MUC1 antibody or an antigen-binding fragment thereof.

As yet another aspect of the present invention, the present invention relates to an immune cell including the chimeric antigen receptor including the anti-MUC1 antibody or an antigen-binding fragment thereof.

In an aspect of the present invention, the immune cell is selected from CAR-T, CAR-NK and CAR-MA.

As yet another aspect of the present invention, the present invention relates to a composition for use in preventing or treating cancer, including one or more of the antibody-drug conjugate, the bispecific antibody, and the chimeric antigen receptor including the anti-MUC1 antibody or an antigen-binding fragment thereof, and the immune cell containing the chimeric antigen receptor.

In an aspect of the present invention, the cancer is selected from the group consisting of skin cancer such as melanoma, liver cancer, hepatocellular carcinoma, hepatocellular carcinoma, stomach cancer, breast cancer, lung cancer, ovarian cancer, bronchial cancer, nasopharyngeal cancer, laryngeal cancer, pancreas cancer, bladder cancer, colorectal cancer, colon cancer, pancreatic cancer, cervical cancer, brain cancer, prostate cancer, bone cancer, skin cancer, thyroid cancer, parathyroid cancer, kidney cancer, esophageal cancer, biliary tract cancer, testicular cancer, rectal cancer, head and neck cancer, cervical spine cancer, ureteral cancer, osteosarcoma, neuroblastoma, fibro sarcoma, rhabdomyosarcoma, astrocytoma, nerve blastoma, glioma, acute myeloid leukemia (AML), acute lymphoblastic leukemia (ALL), adult T-cell leukemia, chronic lymphocytic leukemia (CLL), hairy cell leukemia, myelodysplasia, myeloproliferative disorder, chronic myeloid leukemia (CML), myelodysplastic syndrome (MDS), human T cell leukemia virus type 1 (HTLV-1) leukemia, mastocytosis, acute lymphoblastic leukemia, lymphoma, non-Hodgkin's lymphoma, Hodgkin's lymphoma, multiple myeloma, or solitary myeloma.

As yet another aspect of the present invention, the present invention relates to a composition for use in diagnosing cancer including the anti-MUC1 antibody or an antigen-binding fragment thereof.

As yet another aspect of the present invention, the present invention relates to a method for preparing an anti-MUC1 antibody or an antigen-binding fragment thereof including expressing an expression vector including a nucleic acid encoding the anti-MUC1 antibody or an antigen-binding fragment thereof of in a host cell.

## Claims

1. An anti-MUC1 antibody or an antigen-binding fragment thereof that specifically binds to a polypeptide comprising a contiguous amino acid sequence within a C-terminal extracellular domain of MUC1, and comprises one or more of a heavy chain variable region including a heavy chain CDR1, a heavy chain CDR2, or a heavy chain CDR3; and a light chain variable region including a light chain CDR1, a light chain CDR2, or a light chain CDR3, wherein a lysine (K) residue included in the light chain CDR sequence is substituted with another amino acid.

2. The anti-MUC1 antibody or an antigen-binding fragment thereof of claim 1, wherein the lysine (K) residue included in the light chain CDR sequence is K24 or K30 of the light chain CDR.

3. The anti-MUC1 antibody or an antigen-binding fragment thereof of claim 1, wherein the lysine (K) residue included in the light chain CDR sequence is substituted with an amino acid selected from the group consisting of arginine (R), histidine (H), aspartic acid (D) or glutamic acid (G), glycine (G), alanine (A), valine (V), methionine (M), phenylalanine (F), tyrosine (Y), tryptophan (W), leucine (L), and isoleucine (I).

4. The anti-MUC1 antibody or an antigen-binding fragment thereof of claim 1, wherein the heavy chain variable region includes a heavy chain CDR1 represented by SEQ ID NO: 1, a heavy chain CDR2 represented by SEQ ID NO: 2, or a heavy chain CDR3 represented by SEQ ID NO: 3, and the light chain variable region includes a light chain CDR1 represented by SEQ ID NO: 4, a light chain CDR2 represented by SEQ ID NO: 5, or a light chain CDR3 represented by SEQ ID NO: 6.

5. An antibody-drug conjugate comprising the anti-MUC1 antibody or an antigen-binding fragment thereof of claim 1.

6. A bispecific antibody comprising the anti-MUC1 antibody or an antigen-binding fragment thereof of claim 1.

7. A chimeric antigen receptor (CAR) comprising the anti-MUC1 antibody or an antigen-binding fragment thereof of claim 1.

8. An immune cell comprising the chimeric antigen receptor of claim 7.

9. The immune cell of claim 8, wherein the immune cell is selected from CAR-T, CAR-NK and CAR-MA.

10. A composition for use in preventing or treating cancer, comprising one or more of the antibody-drug conjugate, the bispecific antibody, the chimeric antigen receptor , and the immune cell comprising the chimeric antigen receptor according to any one of claims 5 to 8.

11. The composition for use in preventing or treating cancer of claim 10, wherein the cancer is selected from the group consisting of skin cancer such as melanoma, liver cancer, hepatocellular carcinoma, hepatocellular carcinoma, stomach cancer, breast cancer, lung cancer, ovarian cancer, bronchial cancer, nasopharyngeal cancer, laryngeal cancer, pancreas cancer, bladder cancer, colorectal cancer, colon cancer, pancreatic cancer, cervical cancer, brain cancer, prostate cancer, bone cancer, skin cancer, thyroid cancer, parathyroid cancer, kidney cancer, esophageal cancer, biliary tract cancer, testicular cancer, rectal cancer, head and neck cancer, cervical spine cancer, ureteral cancer, osteosarcoma, neuroblastoma, fibro sarcoma, rhabdomyosarcoma, astrocytoma, nerve blastoma, glioma, acute myeloid leukemia (AML), acute lymphoblastic leukemia (ALL), adult T-cell leukemia, chronic lymphocytic leukemia (CLL), hairy cell leukemia, myelodysplasia, myeloproliferative disorder, chronic myeloid leukemia (CML), myelodysplastic syndrome (MDS), human T cell leukemia virus type 1 (HTLV-1) leukemia, mastocytosis, acute lymphoblastic leukemia, lymphoma, non-Hodgkin's lymphoma, Hodgkin's lymphoma, multiple myeloma, or solitary myeloma.

12. A composition for use in diagnosing cancer comprising the antibody or an antigen-binding fragment thereof of claim 1.

13. A method for preparing an anti-MUC1 antibody or an antigen-binding fragment thereof comprising expressing an expression vector including a nucleic acid encoding the anti-MUC1 antibody or an antigen-binding fragment thereof of claim 1 in a host cell.
